(19)

Europäisches
Patentamt

European
Patent Office

Office européen
des brevets

(11) **EP 3 081 167 A2**

(12) **EUROPEAN PATENT APPLICATION**

(43) Date of publication:
**19.10.2016 Bulletin 2016/42**

(51) Int Cl.:
**A61B 8/08** (2006.01)

(21) Application number: **16165539.4**

(22) Date of filing: **15.04.2016**

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **15.04.2015 US 201562147860 P
26.08.2015 KR 20150120538
05.10.2015 KR 20150139998**

(71) Applicant: **Samsung Medison Co., Ltd.
Hongcheon-gun, Gangwon-do, 25108 (KR)**

(72) Inventors:
• **LEE, Jin-yong
  25108 Gangwon-do (KR)**
• **LEE, Bong-heon
  25108 Gangwon-do (KR)**
• **PARK, Sung-wook
  25108 Gangwon-do (KR)**

• **PARK, Jin-ki
  25108 Gangwon-do (KR)**
• **CHANG, Hyuk-jae
  25108 Gangwon-do (KR)**
• **CHUNG, Nam-sik
  25108 Gangwon-do (KR)**
• **HONG, Geu-ru
  25108 Gangwon-do (KR)**
• **SHIM, Chi-young
  25108 Gangwon-do (KR)**
• **YOON, Ji-hyun
  25108 Gangwon-do (KR)**
• **CHO, In-jeong
  25108 Gangwon-do (KR)**
• **HEO, Ran
  25108 Gangwon-do (KR)**

(74) Representative: **Jacobs, Bart et al
Arnold & Siedsma
Bezuidenhoutseweg 57
2594 AC The Hague (NL)**

---

(54) **ULTRASOUND SYSTEM FOR DISPLAYING STIFFNESS OF BLOOD VESSEL**

(57) An ultrasound system includes a controller controlling generation of an ultrasound image showing a blood vessel based on an ultrasound echo signal obtained from an object including the blood vessel, and a display displaying the ultrasound image, in which the controller determines a first line and a second line spaced apart from each other with an inner space of the blood vessel interposed therebetween and indicating blood vessel walls in the ultrasound image and determines a color based on information about a change in an interval between the first line and the second line, and the display displays a partial area of the ultrasound image with the determined color.

FIG. 1

## Description

BACKGROUND

1. Field

One or more exemplary embodiments relate to a ultrasound system for displaying stiffness of a blood vessel, and more particularly, to an ultrasound system which displays stiffness around a blood vessel by using a certain color so that the stiffness of the blood vessel may be intuitively recognized.

2. Description of the Related Art

Ultrasound diagnosis apparatuses transmit ultrasound signals generated by transducers of a probe to an object and receive echo signals reflected from the object, thereby obtaining at least one image of an internal part of the object (e.g., soft tissues or blood flow). In particular, ultrasound diagnosis apparatuses are used for medical purposes including observation of the interior of an object, detection of foreign substances, and diagnosis of damage to the object. Such ultrasound diagnosis apparatuses provide high stability, display images in real time, and are safe due to the lack of radioactive exposure, compared to X-ray apparatuses. Therefore, ultrasound diagnosis apparatuses are widely used together with other image diagnosis apparatuses including a computed tomography (CT) apparatus, a magnetic resonance imaging (MRI) apparatus, and the like.

SUMMARY

Additional aspects will be set forth in part in the description which follows and, in part, will be apparent from the description, or may be learned by practice of the presented exemplary embodiments.

According to one or more exemplary embodiments, an ultrasound system includes a controller controlling generation of an ultrasound image showing a blood vessel based on an ultrasound echo signal obtained from an object including the blood vessel, and a display displaying the ultrasound image, in which the controller determines a first line and a second line spaced apart from each other with an inner space of the blood vessel interposed therebetween and indicating blood vessel walls in the ultrasound image and determines a color based on information about a change in an interval between the first line and the second line, and the display displays a partial area of the ultrasound image with the determined color.

The ultrasound system may further include a user interface to input a region of interest, wherein the controller determines the first line and the second line to be located in the region of interest.

The controller may determine information about a change in an interval between the first line and the second line by measuring the interval between the first line and the second line for a first time, and the display may maintain the partial area to be displayed with the determined color for a second time.

Each of the first time and the second time may be one electrocardiogram (ECG) cycle or longer.

The controller may determine stiffness of a blood vessel based on a minimum interval and a maximum interval between the first line and the second line for a first time, and a difference between the minimum interval and the maximum interval, and select a color corresponding to a determined stiffness from a color map.

The controller may select a color located at a position relatively close to one end of the color map when the stiffness is relatively high, and a color located at a position relatively close to the other end of the color map when the stiffness is relatively low.

The information about a change in an interval between the first line and the second line may be determined based on information about a change in an interval between a pair of points one of which is from among a plurality of points included in the first line and the other of which is from among a plurality of points included in the second line corresponding to the plurality of points of the first line.

The interval between the first line and the second line may be an average of intervals between pairs of points, and the information about a change in the interval between the first line and the second line may be information about a change in the average.

The partial area may include a first area located from the first line to outside of the blood vessel and a second area located from the second line to the outside of the blood vessel.

A size of the partial area may be determined based on a difference between a position of the first line in a first frame of the ultrasound image and a position of the first line in a second frame of the ultrasound image.

The partial area may have a size determined based on intervals between a plurality of points included in the first line in a first frame of the ultrasound image and a plurality of points included in the first line in a second frame of the ultrasound image, and may be located from the first line to outside of the blood vessel.

The first line may include a plurality of points including a first point, and the display may display a displacement line to indicate a direction and an amount related to a difference between a position of the first point in a first frame of the ultrasound image and a position of the first point in a second frame of the ultrasound image.

According to one or more exemplary embodiments, a method of displaying an ultrasound image includes generating an ultrasound image showing a blood vessel based on an ultrasound echo signal obtained from an object including the blood vessel;

displaying the ultrasound image, determining a first line and a second line spaced apart from each other with an inner space of the blood vessel interposed therebetween and indicating blood vessel walls in the ultrasound image, determining a color based on information about a change in an interval between the first line and the second line, and displaying a partial area of the ultrasound image with the determined color.

The method may further include receiving a user input to input a region of interest, wherein the first line and the second line are located in the region of interest.

The determining of a color may include determining information about a change in an interval between the first line and the second line is determined by measuring the interval between the first line and the second line for a first time, and the displaying of the partial area with the determined color may include maintaining the partial area to be displayed with the determined color for a second time.

Each of the first time and the second time may be one electrocardiogram (ECG) cycle or longer.

The determining of a color may include determining stiffness of a blood vessel based on a minimum interval and a maximum interval between the first line and the second line for a first time, and a difference between the minimum interval and the maximum interval, and selecting a color corresponding to the determined stiffness from a color map.

A color located at a position relatively close to one end of the color map may be selected when the stiffness is relatively high, and a color located at a position relatively close to the other end of the color map may be selected when the stiffness is relatively low.

The information about a change in an interval between the first line and the second line may be determined based on information about a change in an interval between a pair of points one of which is from among a plurality of points included in the first line and the other of which is from among a plurality of points included in the second line corresponding to the plurality of points of the first line.

The interval between the first line and the second line may be an average of intervals between pairs of points, and the information about a change in the interval between the first line and the second line may be information about a change in the average.

The partial area may include a first area located from the first line to outside of the blood vessel and a second area located from the second line to the outside of the blood vessel.

A size of the partial area may be determined based on a difference between a position of the first line in a first frame of the ultrasound image and a position of the first line in a second frame of the ultrasound image.

The partial area may have a size determined based on intervals between a plurality of points included in the first line in a first frame of the ultrasound image and a plurality of points included in the first line in a second frame of the ultrasound image, and may be located from the first line to outside of the blood vessel.

The first line may include a plurality of points including a first point, and the displaying of the partial area with the determined color may include displaying a displacement line to indicate a direction and an amount related to a difference between a position of the first point in a first frame of the ultrasound image and a position of the first point in a second frame of the ultrasound image.

BRIEF DESCRIPTION OF THE DRAWINGS

Exemplary embodiments of the invention now will be described more fully hereinafter with reference to the accompanying drawings, in which reference numerals denote structural elements.

FIG. 1 is a block diagram showing a configuration of an ultrasound diagnosis apparatus according to an exemplary embodiment;
FIG. 2 is a block diagram showing a configuration of a wireless probe according to an exemplary embodiment;
FIG. 3 is a block diagram showing a configuration of an ultrasound system according to an exemplary embodiment;
FIG. 4 is a flowchart of a method of displaying an ultrasound image, according to an exemplary embodiment;
FIG. 5 illustrates a measurement direction to measure a displacement of a blood vessel, according to an exemplary embodiment;
FIG. 6 illustrates a measurement of the displacement and diameter of a blood vessel, according to an exemplary embodiment; and
FIGS. 7A and 7B, FIG. 8, and FIG. 9 illustrate an ultrasound image showing stiffness of a blood vessel or a movement of a blood vessel, according to an exemplary embodiment.

DETAILED DESCRIPTION

The terms used in this specification are those general terms currently widely used in the art in consideration of functions regarding the inventive concept, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used herein have to be defined based on the meaning of the terms together with the description throughout the specification.

Throughout the specification, it will also be understood that when a component "includes" an element, unless there is another opposite description thereto, it should be understood that the component does not exclude another element and may further include another element. In addition, terms such as "... unit", "... module", or the like refer to units that perform at least one function or operation, and the units may be implemented as hardware or software or as a combination of hardware and software.

Throughout the specification, an "ultrasound image" refers to an image of an object, which is obtained using ultrasound waves. Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom is a material having a density, an effective atomic number, and a volume that are approximately the same as those of an organism. For example, the phantom may be a spherical phantom having properties similar to a human body.

Throughout the specification, a "user" may be, but is not limited to, a medical expert, for example, a medical doctor, a nurse, a medical laboratory technologist, or a medical imaging expert, or a technician who repairs medical apparatuses.

Hereinafter, exemplary embodiments will be described in detail with reference to the accompanying drawings.

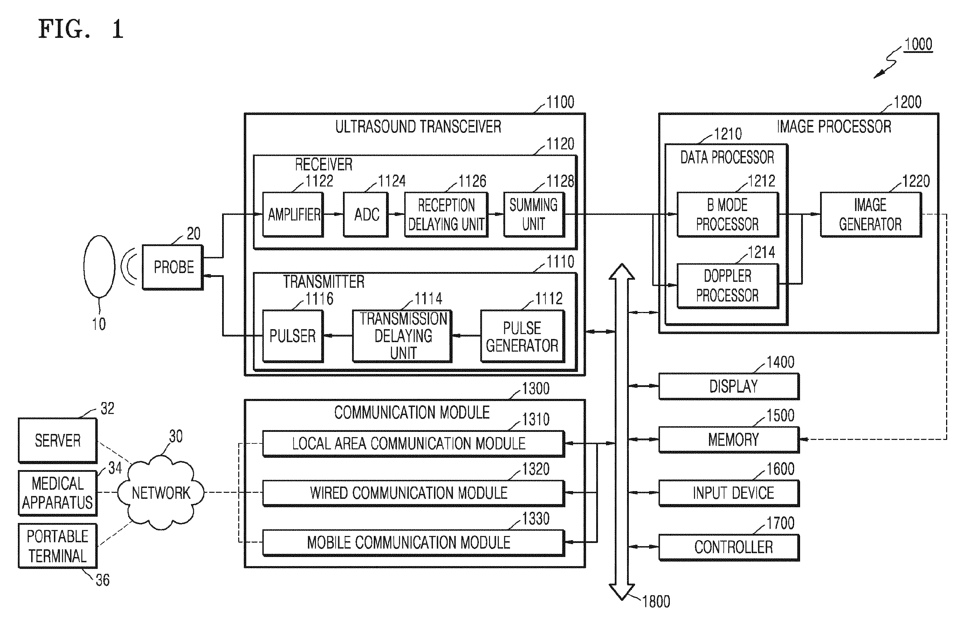

FIG. 1 is a block diagram showing a configuration of an ultrasound diagnosis apparatus 1000 according to an exemplary embodiment. Referring to FIG. 1, the ultrasound diagnosis apparatus 1000 may include a probe 20, an ultrasound transceiver 1100, an image processor 1200, a communication module 1300, a display 1400, a memory 1500, an input device 1600, and a controller 1700, which may be connected to one another via buses 1800.

The ultrasound diagnosis apparatus 1000 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet personal computer (PC).

The probe 20 transmits ultrasound waves to an object 10 in response to a driving signal applied by the ultrasound transceiver 1100 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 1000 by wire or wirelessly, and according to exemplary embodiments, the ultrasound diagnosis apparatus 1000 may include a plurality of probes 20.

A transmitter 1110 supplies a driving signal to the probe 20. The transmitter 110 includes a pulse generator 1112, a transmission delaying unit 1114, and a pulser 1116. The pulse generator 1112 generates pulses for forming transmission ultrasound waves based on a pulse repetition frequency (PRF), and the transmission delaying unit 1114 delays the pulses by delay times necessary for determining transmission directionality. The pulses which have been delayed correspond to a plurality of piezoelectric vibrators included in the probe 20, respectively. The pulser 1116 applies a driving signal (or a driving pulse) to the probe 20 based on timing corresponding to each of the pulses which have been delayed.

A receiver 1120 generates ultrasound data by processing echo signals received from the probe 20. The receiver 120 may include an amplifier 1122, an analog-to-digital converter (ADC) 1124, a reception delaying unit 1126, and a summing unit 1128. The amplifier 1122 amplifies echo signals in each channel, and the ADC 1124 performs analog-to-digital conversion with respect to the amplified echo signals. The reception delaying unit 1126 delays digital echo signals output by the ADC 1124 by delay times necessary for determining reception directionality, and the summing unit 1128 generates ultrasound data by summing the echo signals processed by the reception delaying unit 1126. In some exemplary embodiments, the receiver 1120 may not include the amplifier 1122. In other words, if the sensitivity of the probe 20 or the capability of the ADC 1124 to process bits is enhanced, the amplifier 1122 may be omitted.

The image processor 1200 generates an ultrasound image by scan-converting ultrasound data generated by the ultrasound transceiver 1100. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving velocity of an object as a waveform.

A B mode processor 1212 extracts B mode components from ultrasound data and processes the B mode components. An image generator 1220 may generate an ultrasound image showing signal intensities as brightness based on the extracted B mode components 1212.

Similarly, a Doppler processor 1214 may extract Doppler components from ultrasound data, and the image generator 1220 may generate a Doppler image showing a movement of an object as colors or waveforms based on the extracted Doppler components.

According to an exemplary embodiment, the image generator 1220 may generate a three-dimensional (3D) ultrasound image via volume-rendering with respect to volume data and may also generate an elasticity image by imaging deformation of the object 10 due to pressure. Furthermore, the image generator 1220 may display various pieces of additional information in an ultrasound image by using text and graphics. In addition, the generated ultrasound image may be stored in the memory 1500.

A display 1400 displays the generated ultrasound image. The display 1400 may display not only an ultrasound image, but also various pieces of information processed by the ultrasound diagnosis apparatus 1000 on a screen image via a graphical user interface (GUI). In addition, the ultrasound diagnosis apparatus 1000 may include two or more displays 1400 according to exemplary embodiments.

The communication module 1300 is connected to a network 30 by wire or wirelessly to communicate with an external device or a server. The communication module 1300 may exchange data with a hospital server or another medical apparatus in a hospital, which is connected thereto via a PACS. Furthermore, the communication module 1300 may perform data communication according to the digital imaging and communications in medicine (DICOM) standard.

The communication module 1300 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 1300 may receive information about a diagnosis history or medical treatment schedule of a patient from a server and utilize the received information to diagnose the patient. Furthermore, the communication module 1300 may perform data communication not only with a server or a medical apparatus in a hospital, but also with a portable terminal of a medical doctor or patient.

The communication module 1300 is connected to the network 30 by wire or wirelessly to exchange data with a server 32, a medical apparatus 34, or a portable terminal 36. The communication module 1300 may include one or more components for communication with external devices. For example, the communication module 1300 may include a local area communication module 1310, a wired communication module 1320, and a mobile communication module 1330.

The local area communication module 1310 refers to a module for local area communication within a distance. Examples of local area communication techniques according to an exemplary embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 1320 refers to a module for communication using electric signals or optical signals. Examples of wired communication techniques according to an exemplary embodiment may include communication via a twisted pair cable, a coaxial cable, an optical fiber cable, and an Ethernet cable.

The mobile communication module 1330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 1500 stores various data processed by the ultrasound diagnosis apparatus 1000. For example, the memory 1500 may store medical data related to diagnosis of an object, such as ultrasound data and an ultrasound image that are input or output, and may also store algorithms or programs which are to be executed in the ultrasound diagnosis apparatus 1000.

The memory 1500 may be any of various storage media, e.g., a flash memory, a hard disk drive, electrically erasable programmable read-only memory (EEPROM), etc. Furthermore, the ultrasound diagnosis apparatus 1000 may utilize web storage or a cloud server that performs the storage function of the memory 1500 online.

The input device 1600 refers to a device via which a user inputs data for controlling the ultrasound diagnosis apparatus 1000. The input device 1600 may include hardware components, such as a keypad, a mouse, a touch pad, a touch screen, and a jog switch. However, exemplary embodiments are not limited thereto, and the input device 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

The controller 1700 may control all operations of the ultrasound diagnosis apparatus 1000. In other words, the controller 1700 may control operations among the probe 20, the ultrasound transceiver 1100, the image processor 1200, the communication module 1300, the display 1400, the memory 1500, and the input device 1600 shown in FIG. 1.

All or some of the probe 20, the ultrasound transceiver 1100, the image processor 1200, the communication module 1300, the display 1400, the memory 1500, the input device 1600, and the controller 1700 may be implemented as software modules. Also, at least one of the ultrasound transmission/reception unit 1100, the image processor 1200, and the communication module 1300 may be included in the control unit 1600; however, the inventive concept is not limited thereto.

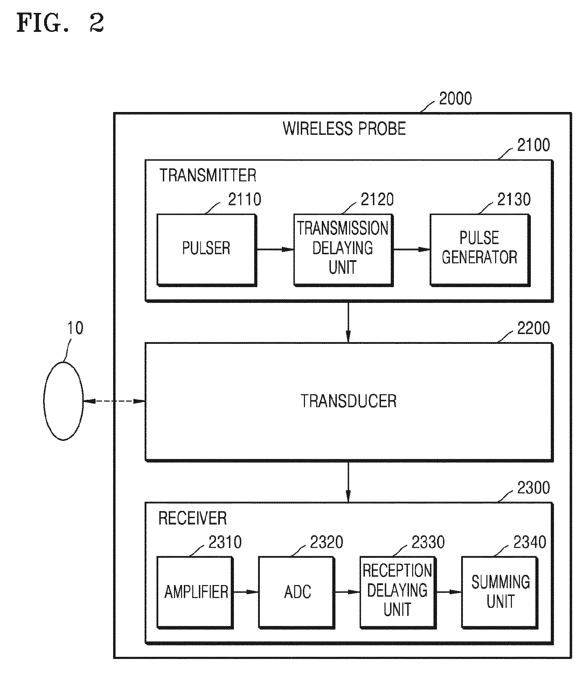

FIG. 2 is a block diagram showing a configuration of a wireless probe 2000 according to an exemplary embodiment. As described above with reference to FIG. 1, the wireless probe 2000 may include a plurality of transducers, and, according to exemplary embodiments, may include some or all of the components of the ultrasound transceiver 100 shown in FIG. 1.

The wireless probe 2000 according to the exemplary embodiment shown in FIG. 2 includes a transmitter 2100, a transducer 2200, and a receiver 2300. Since descriptions thereof are given above with reference to FIG. 1, detailed descriptions thereof will be omitted here. In addition, according to exemplary embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 1.

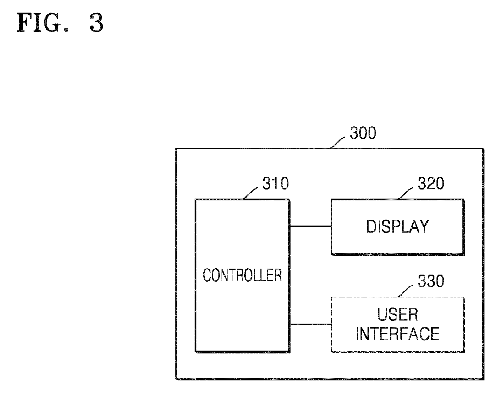

FIG. 3 is a block diagram showing a configuration of an ultrasound system 300 according to an exemplary embodiment. The ultrasound system 300 according to the present exemplary embodiment may be included in the ultrasound diagnosis apparatus 1000 of FIG. 1. A method of displaying an ultrasound image, performed by the ultrasound system 300, may therefore be performed by the ultrasound diagnosis apparatus 1000 of FIG. 1.

According to the present exemplary embodiment, the ultrasound system 300 may include a controller 310 and a display 320. In some exemplary embodiments, the ultrasound system 300 may further include a user interface 330. The ultrasound system 300 may perform some or all of functions performed by the ultrasound diagnosis apparatus 1000 of FIG. 1. For example, the display 320 of the ultrasound system 300 may correspond to the display 1400 of FIG. 1, the controller 310 may include a part of the structure or function of the image processor 1200 and the controller 1700 of FIG. 1. In some exemplary embodiments, the controller 310 may be separated into a plurality of processors to perform the functions of the image processor 1200 and the controller 1700 of FIG. 1, or all functions of the image processor 1200 and the controller 1700 of FIG. 1 may be performed by one processor.

The controller 310 generates an ultrasound image showing a blood vessel based on an ultrasound echo signal obtained from an object including the blood vessel. The ultrasound echo signal may be received through an ultrasound transceiver (not shown) connected to the ultrasound system 300 or may be read out from a memory (not shown) of the ultrasound system 300. The blood vessel may be a carotid artery and a blood vessel ultrasound image may be a B ultrasound image, but the present disclosure is not limited thereto. The controller 310 controls the display 320 to display a generated ultrasound image.

The ultrasound image generated by the controller 310 and displaying a blood vessel may be used to measure a parameter related to a blood vessel. The parameter related to a blood vessel may include at least one of the size, length, and displacement of a blood vessel, the diameter of a blood vessel, stiffness of a blood vessel, a relaxation ratio of a blood vessel, and a movement velocity of a blood vessel wall.

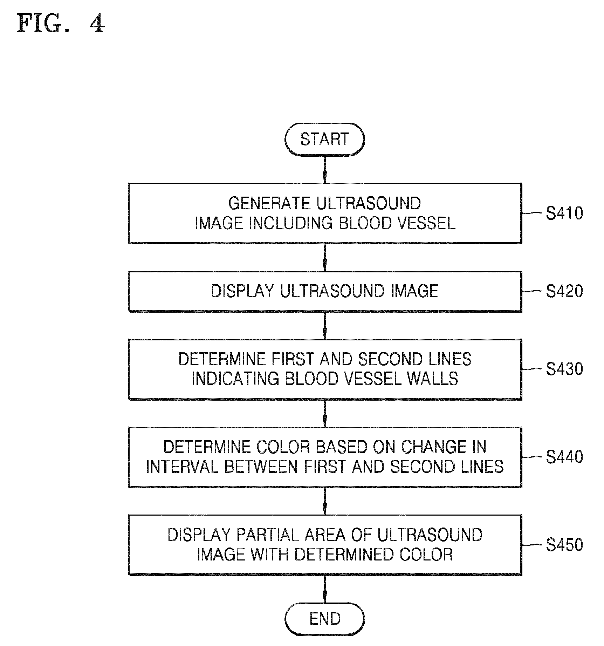

FIG. 4 is a flowchart of a method of displaying an ultrasound image, according to an exemplary embodiment.

In S410, the ultrasound system 300 generates an ultrasound image showing a blood vessel based on an ultrasound echo signal obtained from an object including the blood vessel.

In S420, the ultrasound system 300 displays a generated ultrasound image.

In S430, the ultrasound system 300 determines a first line and a second line indicating blood vessel walls with an inner space of the blood vessel interposed therebetween. The first and second lines may be determined to indicate an entire blood vessel wall displayed in the ultrasound image or only a blood vessel wall located in a particular region of interest (ROI). Also, the first and second lines may be automatically determined as the ultrasound system 300 performs image processing on the ultrasound image or may be a linear line or a curved line directly input by a user. In some exemplary embodiments, the user may set only an ROI through a user input and the ultrasound system 300 may automatically determine the first and second lines in a set ROI.

In S440, the ultrasound system 300 determines a particular color based on information about a change in an interval between the first line and the second line.

In S450, the ultrasound system 300 displays a partial area of an ultrasound image with a determined color.

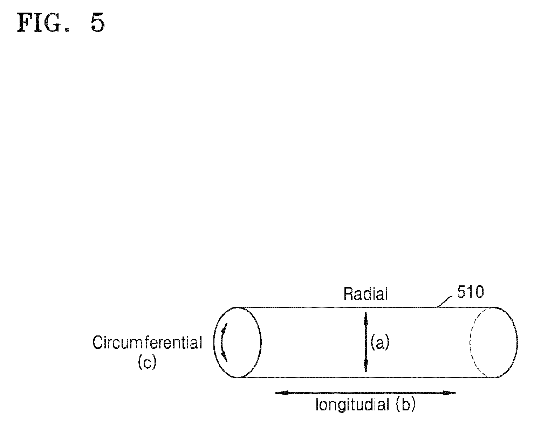

Referring to FIG. 5, in some exemplary embodiments, parameters related to a blood vessel may be measured or determined in relation to a longitudinal direction (b), a radial direction (a), and a circumferential direction (c) of a blood vessel in a blood vessel ultrasound image. An ultrasound image showing a blood vessel may be measured or determined by using a long axis view showing a blood vessel in a longitudinal direction thereof or a short axis view showing a blood vessel in a radial or circumferential direction thereof, and parameters related to a blood vessel may be measured or determined by using the long axis view or the short axis view.

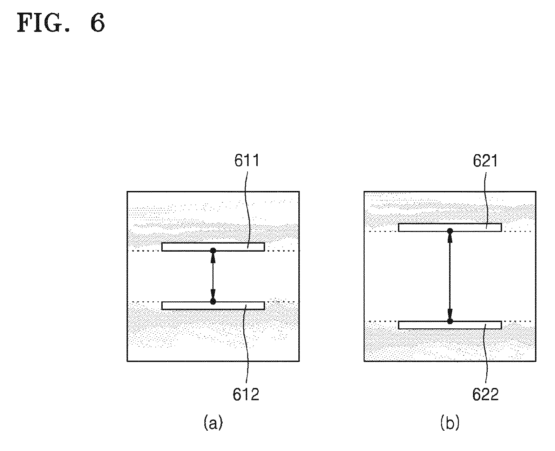

Referring to FIG. 6, the controller 310 may determine a first line 611 (621) and a second line 612 (622) indicating blood vessel walls in an ultrasound image displaying the blood vessel in a long axis view. The first and second lines 611 and 612 (621 and 622) indicating the blood vessel walls may be automatically determined through image processing in the ultrasound system 300 or may be determined by a direct input of a user. According to an exemplary embodiment, the ultrasound system 300 may determine the first and second lines 611 and 612 (621 and 622) by receiving an input of a measurement area or an ROI by a user through the user interface 330 and perform image processing on a blood vessel wall located in the input measurement area or ROI. Alternatively, the first and second lines 611 and 612 (621 and 622) may be received directly from a user through the user interface 330. In addition, although in the exemplary embodiment of FIG. 6 the first and second lines 611 and 612 (621 and 622) are determined to indicate a part of a blood vessel displayed in the ultrasound images (a) and (b), the first and second lines 611 and 612 (621 and 622) may be determined to indicate the entire blood vessel displayed in the ultrasound images (a) and (b).

An image of a blood vessel displayed on the display 320 changes dynamically according to a movement of blood in the blood vessel. For example, the diameter of a blood vessel when a pressure of blood passing through the blood vessel is relatively high is generally larger than the diameter of a blood vessel when a pressure of blood passing through a blood vessel is relatively low. As seen from the ultrasound images, the diameter of a blood vessel displayed in the ultrasound image (b) of FIG. 6 is larger than the diameter of a blood vessel displayed in the ultrasound image (a).

The diameter of a blood vessel may be determined using various methods. For example, the ultrasound system 300 may determine an interval between the first and second lines 611 and 612 in FIG. 6 to be the diameter of a blood vessel. According to an exemplary embodiment, the ultrasound system 300 may determine a distance between a particular point included in the first line 611 and a particular point included in the second line 612 at a particular frame of an ultrasound image, to be a distance between the first and second lines 611 and 612. According to another exemplary embodiment, among points on the first line 611 and points on the second line 612 corresponding to the points on the first line 611, intervals between pairs of points are measured and an average of the measured intervals is calculated. A calculated average value may be determined to be the interval between the first and second lines 611 and 612. In this regard, some or all points included in the first line 611 and some or all points included in the second line 612 may match each other one-to-one. In some exemplary embodiments, the first and second lines 611 and 612 may include the same number of points. However, in some exemplary embodiments, the first and second lines 611 and 612 may further include points other than the matching points.

Alternatively, when the diameter of a blood vessel changes according to a movement of blood passing through the blood vessel, a degree of change in the diameter of a blood vessel may vary according to the state of the blood vessel. For example, an unhealthy blood vessel may have higher stiffness than a healthy blood vessel. Having higher stiffness may signify being harder or having lower flexibility. In this case, a degree of change in the diameter of a blood vessel having relatively high stiffness may be lower than a degree of change in the diameter of a blood vessel having relatively low stiffness. For example, when the ultrasound image (a) of FIG. 6 is an image when a blood vessel contracts at its maximum, and the ultrasound image (b) of FIG. 6 is an image when a blood vessel is relaxed at its maximum, as stiffness of a blood vessel increases, a difference in the diameter between the ultrasound image (a) and the ultrasound image (b) may relatively decrease.

Accordingly, a user may determine a degree of stiffness of a blood vessel by directly identifying a degree of change in the diameter of a blood vessel. However, the above-described method of determining stiffness by directly identifying a degree of change in the diameter of a blood vessel may have low accuracy and may be inconvenient.

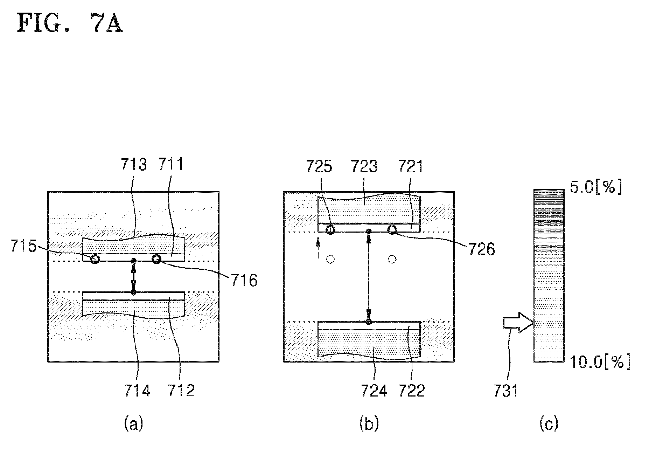

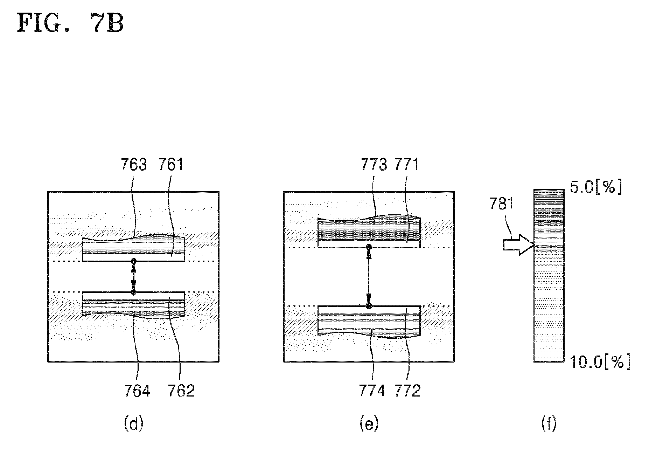

FIGS. 7A and 7B illustrate ultrasound images which may facilitate identification of stiffness of a blood vessel, according to an exemplary embodiment.

An ultrasound image (a) and an ultrasound image (b) of FIG. 7A are respectively a first frame (a) and a second frame (b) of an ultrasound image including a blood vessel. For convenience of explanation, an appropriate blood vessel is referred to as a target blood vessel. The second frame (b) may be a frame that is displayed just next to the first frame (a) or a frame that is displayed after a few frames.

The ultrasound system 300 determines a first line 711 and a second line 712 located with an inner space of the target blood vessel interposed therebetween at a first frame (a) of an ultrasound image, and displays the first and second lines 711 and 712 on the display 320 with the first frame (a). In this connection, an interval between the first and second lines 711 and 712 varies according to a flow of blood in the blood vessel. For convenience of explanation, the interval between the first and second lines 711 and 712 may be referred to as a diameter of interest of the target blood vessel. The diameter of interest may be determined using various methods as described above with reference to FIG. 6. In general, when a pressure of blood passing through a blood vessel periodically changes according to a heart beat, a diameter of interest of the target blood vessel may repeatedly increase or decrease.

The ultrasound system 300 measures an interval between the first and second lines 711 and 712 at a first frame, obtains information about a change in the interval between the first and second lines 711 and 712, and determines stiffness of a blood vessel based on the information. According to an exemplary embodiment, the ultrasound system 300 measures a minimum interval and a maximum interval between the first and second lines 711 and 712 for a time, and determines a ratio of a difference between the maximum interval and the minimum interval with respect to the minimum interval. For convenience of explanation, the ratio is referred to as a relaxation ratio. The time may be one ECG cycle or longer.

For example, it is assumed that the first frame (a) of an ultrasound image for a time is a frame when the diameter of interest is at a minimum, for example, 5 mm, and that the first frame (b) of an ultrasound image for a time is a frame when the diameter of interest is at a maximum, for example, 5.4 mm. The ultrasound system 300 may determine that a relaxation rate is 8% according to the following equation.

$$\text{Relaxation Rate} = (5.4 - 5.0)/5.0 = 8\%$$

However, the relaxation rate may be determined using various methods in addition to the above equation. The ultrasound system 300 may employ any method so long as a high relaxation rate is determined when stiffness of a blood vessel is low and flexibility is high so that relaxation occurs easily.

It may be said that the higher the relaxation rate, the lower the stiffness, and the lower the relaxation rate, the higher the stiffness. The stiffness may be defined by one of various methods using a relaxation rate or directly using a change value of a diameter. For example, the stiffness may be defined as follows.

$$\text{Stiffness} = 100\% - \text{Relaxation Rate}$$

When the stiffness is defined as above, stiffness in the above example is 92%.

In some exemplary embodiments, the ultrasound system 300 may determine an appropriate color from a color map (c) of FIG. 7A based on a determined stiffness or relaxation rate. Determining a color from the color map (c) based on a determined stiffness may be essentially the same as determining a color from the color map (c) based on a determined relaxation rate. For example, when a color corresponding to a relaxation rate of 10% is located at one end in a lower side of the color map (c) and a color corresponding to a relaxation rate of 5% is located at the opposite end in the color map (c), in the above example, the ultrasound system 300 may select a color 731 corresponding to a relaxation rate of 8% located somewhere between the opposite ends of the color map (c).

Next, the ultrasound system 300 displays a partial area, for example, areas 713 and 714, of an ultrasound image with a determined color. The determined color may be displayed to be partially transparent so as to overlap with the partial areas of an ultrasound image.

In FIG. 7B, a first frame (d) of an ultrasound image and a second frame (e) of an ultrasound image show a blood vessel having high stiffness compared to the exemplary embodiment of FIG. 7A. For example, in the blood vessel of FIG. 7B, it is assumed that the first frame (d) is a frame when a diameter of interest is the minimum, for example, 5 mm, and that the second frame (e) is a frame when a diameter of interest is the maximum, for example, 5.3 mm. When using the above-described equation, the ultrasound system 300 may determine that a relaxation rate is 6% and stiffness is 94%.

$$\text{Relaxation Rate} = (5.3 - 5.0)/5.0 = 6\%$$

$$\text{Stiffness} = 100\% - \text{Relaxation Rate}$$

In this case, the ultrasound system 300 may determine a color corresponding to the determined relaxation rate or stiffness of FIG. 7B, from a color map (f). For example, when a color corresponding to a relaxation rate of 10% is located at one end in a lower side of the color map (f) and a color corresponding to a relaxation rate of 5% is located at the opposite end in the color map (f), in the above example, the ultrasound system 300 may select a color 781 corresponding to a relaxation rate of 6% located somewhere between the opposite ends of the color map (f).

Accordingly, in the case of FIGS. 7A and 7B, a user may intuitively recognize how high stiffness or a relaxation rate of a target blood vessel is through a color displayed in a partial area around the blood vessel. For user convenience, as in the cases in FIGS. 7A and 7B, the color maps (c) and (f) may be displayed with the ultrasound images (a), (b), (d), and (e).

In some exemplary embodiments, the ultrasound system 300 may maintain an appropriate partial area to be displayed with a determined color for a time. The time may be one ECG cycle or longer. As the user recognizes the color that is maintained to be displayed in the appropriate area for the time, stiffness of a target blood vessel may be easily determined compared to a case in which the appropriate color is continuously changed.

In some exemplary embodiments, the partial area displayed with the determined color may include at least one of a first area corresponding to the first line and a second area corresponding to the second line in an ultrasound image. The first area may be displayed above the first line. For example, when the first frame (a) of the ultrasound image in FIG. 7A is referred to, the first area may be the area 713 including an area from the first line 711 to a position separated a distance from the first line 711 to outside of the blood vessel. In a similar method, the second area may be the area 714 including an area from the second line 712 to a position separated a distance from the second line 712 to the outside of the blood vessel.

Alternatively, when the ultrasound image is changed according to a movement of a blood vessel, that is, the positions of the first and second lines indicating blood vessel walls are changed, the first and second areas may be changed corresponding to the change of the positions of the first and second lines. For example, in the first frame (a) of the ultrasound image, the first area 713 may be displayed in an area starting from the position of the first line 711 in a state in which the blood vessel is contracted, whereas in the second frame (b) of the ultrasound image, a first area 723 may be displayed in an area starting from the position of the first line 721 in a state in which the blood vessel is relaxed.

Also, the ultrasound system 300 may determine the size of the first area based on a displacement of the first line. In other words, when the displacement of the first line is relatively large, the size of the first area may be determined to be relatively large. For example, in FIG. 7A, the ultrasound system 300 may determine the size of the first area of the second frame (b) such that the size of the first area is proportional to a displacement between the first line of the first frame (a) and the first line of the second frame (b) of the ultrasound image. Comparing the case of FIG. 7A and the case of FIG. 7B, the displacement of the first line in the case of FIG. 7A is larger than the displacement in the case of FIG. 7B. Accordingly, the size of the first area 723 in the second frame (b) of FIG. 7A is larger than the size of a first area 773 in the second frame (e) of FIG. 7B.

In some exemplary embodiments, in the measurement of the displacement of the first line, the ultrasound system 300 may determine the displacement of the first line by comparing the position of the first line in a frame when the diameter of a target blood vessel is the minimum and the position of the first line in a frame when the diameter of a target blood vessel is the maximum. The ultrasound system 300 may determine the size of the first area according to the determined displacement of the first line.

In another exemplary embodiment, in the determination of the size of the first area displayed on a current frame of an ultrasound image, the ultrasound system 300 may compare the position of the first line in the current frame and the position of the first line in a previous frame and determine the size of the first area according to a displacement as a result of the comparison. In this case, the size of the first area is dynamically changed according to a movement of a blood vessel wall in the ultrasound image. In particular, when stiffness of a target blood vessel is low, a degree or velocity of a dynamic change in the size of the first area is further increased. The user may recognize that stiffness of a target blood vessel is relatively low or a relaxation rate is relatively high, through a visual effect showing that the degree or velocity of a dynamic change in the size of the first area is relatively large.

In another exemplary embodiment, in the determination of the size of the first area displayed in a current frame of an ultrasound image, the ultrasound system 300 may determine the size of the first area based on a displacement of a plurality of points included in the first line. For convenience of explanation, appropriate points are referred to as a plurality of tracking points. In other words, when a displacement of a particular tracking point included in the first line is relatively large, a portion of the first area corresponding to the particular tracking point may be determined to be located far from the particular tracking point. For example, assuming that, in FIG. 7A, tracking points 715 and 725 and tracking points 716 and 726 are included in the first line, and that the first frame and the second frame are compared with each other, if a displacement between the tracking points 716 and 726 is larger than a displacement between the tracking points 715 and 725, the sizes of the first areas 713 and 723 may be determined such that a portion corresponding to the tracking points 716 and 726 is located farther from the first line than a portion corresponding to the tracking points 715 and 725.

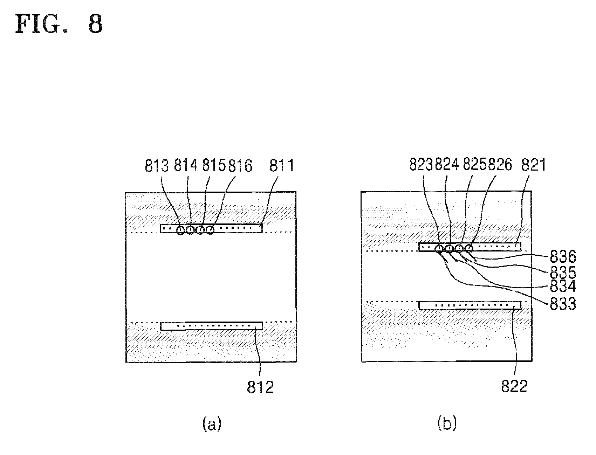

FIG. 8 illustrates an ultrasound image displaying a movement of a blood vessel wall, according to an exemplary embodiment.

In FIG. 8, when comparing a first frame (a) and a second frame (b) of an ultrasound image, in the first frame (a), four tracking points 813, 814, 815, and 816 included in the first line are moved to four other points 823, 824, 825, and 826 in the second frame (b). The first line in the first frame (a) may include more tracking points in addition to the four tracking points 813, 814, 815, and 816. The ultrasound system 300 may display a direction and an amount of a changed position of each point in the second frame (b) by using a particular point or line. For example, displacement lines 833, 834, 835, and 836 are respectively displayed with respect to the points 823, 824, 825, and 826 on the second line. The displacement line denotes changed distance and direction when the points 823, 824, 825, and 826 are compared with the tracking points in the first frame. When the displacement line is displayed to be relatively long, it signifies that a changed distance is relatively large.

In some exemplary embodiments, considering that a changed amount of the position of a tracking point is relatively small, an amount of a change in the position of a tracking point is enlarged several times and an enlarged displacement line is displayed in an appropriate direction and thus the user may easily recognize the direction and amount of the displacement of the tracking point.

Also, in some exemplary embodiments, the displacement of FIG. 8 may be displayed together with the partial areas, for example, the first area and the second area, of FIGS. 7A and 7B.

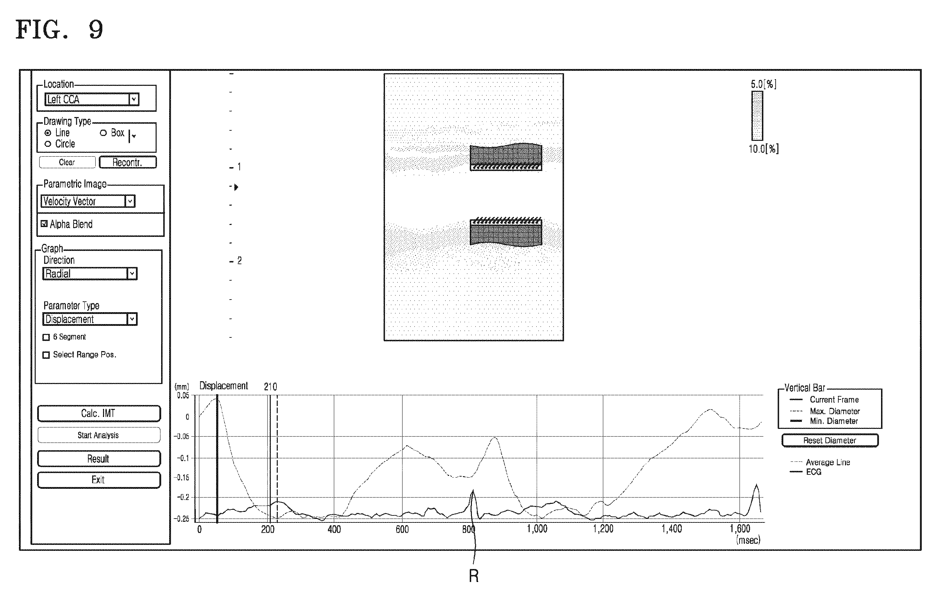

FIG. 9 illustrates an image displaying a displacement graph of a blood vessel with a movement of a wall of the blood vessel, according to an exemplary embodiment.

A dotted graph, that is, an average line, of graphs shown in a lower end of the image of FIG. 9 indicates an average of intervals between a plurality of points included in the first line and the second line set in a blood vessel ultrasound image in an upper portion of the image, according to a passage of time. For convenience of explanation, the average of the intervals between the points included in the first line and the second line may be referred to as a diameter of a target blood vessel. Since a process of determining the average of the intervals between the points corresponding to each other and included in the first line and the second line has already been described in relation to FIG. 6, a description of the method is omitted here.

In the dotted graph, when a displacement value at a start point is 0, that is, a diameter at the point is a reference diameter value, if the displacement value changes in a positive (+) direction, it signifies that the target blood vessel contracts and thus the diameter of the target blood vessel becomes smaller than the reference diameter value. In contrast, when the displacement value changes in a negative (-) direction, it signifies that the target blood vessel expands and thus the diameter of the target blood vessel becomes larger than the reference diameter value. In detail, the minimum diameter of the target blood vessel is smaller than the reference diameter value by about 0.05 mm and the maximum diameter is larger than the reference diameter value by about 0.25 mm. Accordingly, a difference between the minimum diameter of the target blood vessel and the maximum diameter of the target blood vessel may be about 0.3 mm.

The ultrasound system 300 may determine a color displayed in an area corresponding to the first and second lines of the target blood vessel, by using the above obtained difference between the minimum diameter and the maximum diameter. In detail, the ultrasound system 300 may determine a ratio of the difference between the minimum diameter and the maximum diameter with respect to the reference diameter value of the target blood vessel, and determine a particular color from a color map according to the determined ratio.

The ultrasound system and the method of displaying an ultrasound image according to the exemplary embodiments can also be embodied as computer-readable codes on a non-transitory computer-readable recording medium. The non-transitory computer-readable recording medium is any data storage device that can store data which can thereafter be read by a computer system. Examples of the non-transitory computer-readable recording medium include read-only memory (ROM), random-access memory (RAM), CD-ROMs, magnetic tapes, floppy disks, optical data storage devices, etc. The non-transitory computer-readable recording medium can also be distributed over network coupled computer systems so that the computer-readable code is stored and executed in a distributive manner.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope as defined by the following claims.

**Claims**

1.   An ultrasound system comprising:

a controller controlling generation of an ultrasound image showing a blood vessel based on an ultrasound echo signal obtained from an object including the blood vessel; and
a display displaying the ultrasound image, wherein the controller determines a first line and a second line spaced apart from each other with an inner space of the blood vessel interposed therebetween and indicating blood vessel walls in the ultrasound image and determines a color based on information about a change in an interval between the first line and the second line, and
wherein the display displays a partial area of the ultrasound image with the determined color.

2.   The ultrasound system of claim 1, further comprising a user interface to input a region of interest, wherein the controller determines the first line and the second line to be located in the region of interest.

3.   The ultrasound system of claim 1 or 2, wherein the controller determines information about a change in an interval between the first line and the second line by measuring the interval between the first line and the second line for a first time, and the display maintains the partial area to be displayed with the determined color for a second time.

4.   The ultrasound system of claim 3, wherein each of the first time and the second time is one electrocardiogram (ECG) cycle or longer.

5.   The ultrasound system of any of the previous claims, wherein the controller determines stiffness of a blood vessel based on a minimum interval and a maximum interval between the first line and the second line for a first time, and a difference between the minimum interval and the maximum interval, and selects a color corresponding to a determined stiffness from a color map.

6.   The ultrasound system of any of the previous claims, wherein the information about a change in an interval between the first line and the second line is determined based on information about a change in an interval between a pair of points one of which is from among a plurality of points included in the first line and the other of which is from among a plurality of points included in the second line corresponding to the plurality of points of the first line.

7.   The ultrasound system of any of the previous claims, wherein a size of the partial area is determined based on a difference between a position of the first line in a first frame of the ultrasound image and a position of the first line in a second frame of the ultrasound image.

8.   A method of displaying an ultrasound image, the method comprising:

generating an ultrasound image showing a blood vessel based on an ultrasound echo signal obtained from an object including the blood vessel;

displaying the ultrasound image;

determining a first line and a second line spaced apart from each other with an inner space of the blood vessel interposed therebetween and indicating blood vessel walls in the ultrasound image;

determining a color based on information about a change in an interval between the first line and the second line; and

displaying a partial area of the ultrasound image with the determined color.

9. The method of claim 8, further comprising receiving a user input to input a region of interest, wherein the first line and the second line are located in the region of interest.

10. The method of claim 8 or 9, wherein the determining of a color comprises determining information about a change in an interval between the first line and the second line is determined by measuring the interval between the first line and the second line for a first time, and

the displaying of the partial area with the determined color comprises maintaining the partial area to be displayed with the determined color for a second time.

11. The method of claim 10, wherein each of the first time and the second time is one electrocardiogram (ECG) cycle or longer.

12. The method of any of the claims 8-11, wherein the determining of a color comprises:

determining stiffness of a blood vessel based on a minimum interval and a maximum interval between the first line and the second line for a first time, and a difference between the minimum interval and the maximum interval; and

selecting a color corresponding to the determined stiffness from a color map.

13. The method of any of the claims 8-12, wherein the information about a change in an interval between the first line and the second line is determined based on information about a change in an interval between a pair of points one of which is from among a plurality of points included in the first line and the other of which is from among a plurality of points included in the second line corresponding to the plurality of points of the first line.

14. The method of any of the claims 8-138, wherein the partial area comprises a first area located from the first line to outside of the blood vessel and a second area located from the second line to the outside of the blood vessel.

15. The method of any of the claims 8-14, wherein a size of the partial area is determined based on a difference between a position of the first line in a first frame of the ultrasound image and a position of the first line in a second frame of the ultrasound image.

FIG. 1

FIG. 2

WIRELESS PROBE 2000

TRANSMITTER 2100

PULSER 2110 → TRANSMISSION DELAYING UNIT 2120 → PULSE GENERATOR 2130

10

TRANSDUCER 2200

RECEIVER 2300

AMPLIFIER 2310 → ADC 2320 → RECEPTION DELAYING UNIT 2330 → SUMMING UNIT 2340

FIG. 3

FIG. 4

```
                    ┌─────────┐
                    │  START  │
                    └─────────┘
                         │
                         ▼
        ┌───────────────────────────────────┐
        │        GENERATE ULTRASOUND         │
        │    IMAGE INCLUDING BLOOD VESSEL    │─── S410
        └───────────────────────────────────┘
                         │
                         ▼
        ┌───────────────────────────────────┐
        │       DISPLAY ULTRASOUND IMAGE     │─── S420
        └───────────────────────────────────┘
                         │
                         ▼
        ┌───────────────────────────────────┐
        │    DETERMINE FIRST AND SECOND LINES│
        │      INDICATING BLOOD VESSEL WALLS │─── S430
        └───────────────────────────────────┘
                         │
                         ▼
        ┌───────────────────────────────────┐
        │   DETERMINE COLOR BASED ON CHANGE IN│
        │  INTERVAL BETWEEN FIRST AND SECOND LINES│─── S440
        └───────────────────────────────────┘
                         │
                         ▼
        ┌───────────────────────────────────┐
        │  DISPLAY PARTIAL AREA OF ULTRASOUND│
        │    IMAGE WITH DETERMINED COLOR     │─── S450
        └───────────────────────────────────┘
                         │
                         ▼
                    ┌─────────┐
                    │   END   │
                    └─────────┘
```

FIG. 5

FIG. 6

(a)                    (b)

FIG. 7A

(a)                    (b)                    (c)

FIG. 7B

763 761

773 771

781

5.0[%]

10.0[%]

764 762

774 772

(d)

(e)

(f)

FIG. 8

(a)                    (b)

# FIG. 9